# EUROPEAN PATENT APPLICATION

(11) **EP 4 534 544 A1**
(43) Date of publication of application: **09.04.2025**
(21) Application number: 23816364.6
(22) Date of filing: 31.05.2023
(51) Int. Cl.: C07K 5/072, A61K 38/05, A61P 27/02

(54) **NOVEL D-AMINO ACID DERIVATIVE AND PHARMACEUTICAL COMPOSITION COMPRISING SAME**

(30) Priority: 31.05.2022 KR 20220066477
(71) Applicant: Senelix Co., Ltd., Seoul 05836 (KR)
(72) Inventor: HAHN, Jang-Hee, Chuncheon-si Gangwon-do 24375 (KR); KIM, Min-Seo, Chuncheon-si Gangwon-do 24371 (KR); JU, Hyun-Mi, Wonju-si Gangwon-do 26324 (KR); KIM, Bong-Cheol, Seongnam-si Gyeonggi-do 13525 (KR); KIM, Dong-Sik, Seoul 08750 (KR)
(74) Representative: V.O.
(86) International application number: PCT/KR2023/007448
(87) International publication number: WO 2023/234703

(57) **Abstract**

The present disclosure provides a novel peptide derivative containing a specific D-amino acid, and a pharmaceutical composition for preventing, ameliorating or treating ophthalmic diseases containing the same. The peptide derivative of the present disclosure may exhibit excellent anti-inflammatory activity and also show excellent improvement activity in dry eye syndrome and corneal damage models. Therefore, it may be effectively useful for preventing, ameliorating and treating various eye diseases.

## Description

### [Technical Field]

The present disclosure relates to a novel D-amino acid derivative and a pharmaceutical composition containing the same. More specifically, the present disclosure relates to a peptide derivative including a novel specific D-amino acid, and a pharmaceutical composition containing the same for preventing, ameliorating or treating ophthalmic diseases.

### [Background Art]

Dry eye syndrome (DES), which is one of the most common ophthalmic diseases, is the condition of having dry eyes. Dry eye syndrome occurs when the eye doesn't produce enough tears or the tears are evaporated too quickly due to instability of the tear film. Dry eye syndrome is caused by various causes, such as contact lens use, meibomian gland dysfunction, etc. Artificial tears are usually used to ameliorate dry eye syndrome, and cyclosporin-containing preparations (e.g., Restasis^{®}) are used for the treatment of dry eye syndrome.

Ocular inflammation includes inflammation of the eye, such as conjunctivitis, keratitis, keratoconjunctivitis, uveitis, scleritis, etc. Ocular inflammation is known to be caused by a variety of causes. For example, dry eye syndrome is known to cause keratoconjunctivitis, etc., and cyclosporine-containing preparations (e.g., Restasis^{®}) are also used for the treatment of the ocular inflammation associated with keratoconjunctivitis. Various therapies are being used for ocular inflammation depending on the symptoms and the cause of the disease. For example, antibiotics are used to treat bacterial keratitis/conjunctivitis, antihistamines are used to treat allergic keratitis/conjunctivitis, and steroids are used to treat uveitis.

Conjunctival and/or corneal injuries refer to damages or injuries to the conjunctiva and/or cornea caused by a variety of causes, including heat, chemical burns, etc. These damages lead to corneal ulcer or ulcerative keratitis. For ocular inflammation caused by conjunctival and/or corneal damage, various therapies are used depending on the symptoms and causes of the disease. However, there is a need for the development of drugs with satisfactory activity for amelioration or treatment of conjunctival/corneal injuries and corneal ulcer themselves.

Ophthalmic diseases such as dry eye syndrome, ocular inflammation (e.g., keratitis, conjunctivitis, keratoconjunctivitis, uveitis, scleritis, etc.), conjunctival/corneal injury, corneal ulcer, etc. require long-term treatment and have high recurrence rates. Therefore, the development of a new mechanism of action for maintenance of eye homeostasis, such as effective stabilization of the tear film, suppression of inflammation of the cornea and conjunctiva, and promotion of ocular wound recovery, is required.

### [Disclosure]

### [Technical Problem]

The inventors of the present disclosure have conducted various studies to develop peptide derivatives that can ameliorate or treat ophthalmic diseases, including dry eye syndrome, ocular inflammation and conjunctival and/or corneal damage. In particular, the inventors of the present disclosure have selected D-amino acid-containing peptide derivatives that exhibit excellent anti-inflammatory activity in tests using cell lines and conducted various studies. As a result, it was found that peptide derivatives containing specific D-amino acids exhibit excellent improvement activity in dry eye syndrome and corneal damage models.

Thus, the present disclosure is directed to providing a peptide derivative containing the specific D-amino acid.

In addition, the present disclosure is directed to providing a pharmaceutical composition for preventing, ameliorating or treating ophthalmic diseases, which contains a peptide derivative containing the specific D-amino acid.

In addition, the present disclosure is directed to providing a use of a peptide derivative containing the specific D-amino acid in therapy.

In addition, the present disclosure is directed to providing a use of a peptide derivative containing the specific D-amino acid for use in the prevention or treatment of ophthalmic diseases.

In addition, the present disclosure is directed to providing a method for preventing or treating an ophthalmic disease in a subject in need thereof, which includes administering a peptide derivative containing the specific D-amino acid to the subject.

### [Technical Solution]

In accordance with one aspect of the present disclosure, there is provided a compound of Chemical Formula 1 or a pharmaceutically acceptable salt thereof: 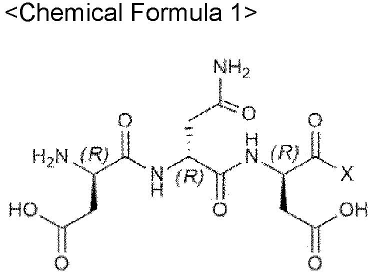 wherein X is -OH or -NH₂.

In the compound of the present disclosure, or a pharmaceutically acceptable salt thereof, X may be specifically -NH₂.

In accordance with another aspect of the present disclosure, there is provided a pharmaceutical composition for preventing, ameliorating or treating ophthalmic disease, which contains the compound described above or a pharmaceutically acceptable salt thereof.

In accordance with another aspect of the present disclosure, there is provided a cosmetic composition or a cosmetic additive for preventing or ameliorating an ophthalmic disease, which contains the compound or a pharmaceutically acceptable salt thereof.

In accordance with another aspect of the present disclosure, there is provided a use of the compound or a pharmaceutically acceptable salt thereof in therapy.

In accordance with another aspect of the present disclosure, there is provided a use of the compound or a pharmaceutically acceptable salt thereof for use in the prevention or treatment of an ophthalmic disease.

In accordance with another aspect of the present disclosure, there is provided a method for preventing or treating an ophthalmic disease in a subject in need thereof, which includes administering the compound or a pharmaceutically acceptable salt thereof to the subject.

In the present disclosure, the ophthalmic disease may be one or more diseases selected from the group consisting of dry eye syndrome, ocular inflammation, conjunctival damage, corneal damage and corneal ulcer. In an exemplary embodiment, the ophthalmic disease may be dry eye syndrome. In another exemplary embodiment, the ophthalmic disease may be ocular inflammation. In another exemplary embodiment, the ophthalmic disease may be conjunctival damage, corneal damage or corneal ulcer.

The pharmaceutical composition of the present disclosure may be an eye drop formulation. The eye drop formulation may be in the form of a solution or a suspension.

### [Advantageous Effects]

It has been shown by the present disclosure that a peptide derivative containing the D-amino acid (i.e., the compound of Chemical Formula 1) or a pharmaceutically acceptable salt thereof exhibits excellent anti-inflammatory activity and also exhibits excellent improvement activity in dry eye syndrome and corneal damage models. Thus, the compound according to the present disclosure, or a pharmaceutically acceptable salt thereof, may be used for preventing, ameliorating and treating various ophthalmic diseases, e.g., dry eye syndrome, ocular inflammation, conjunctival damage, corneal damage, corneal ulcer, etc.

### [Brief Description of Drawings]

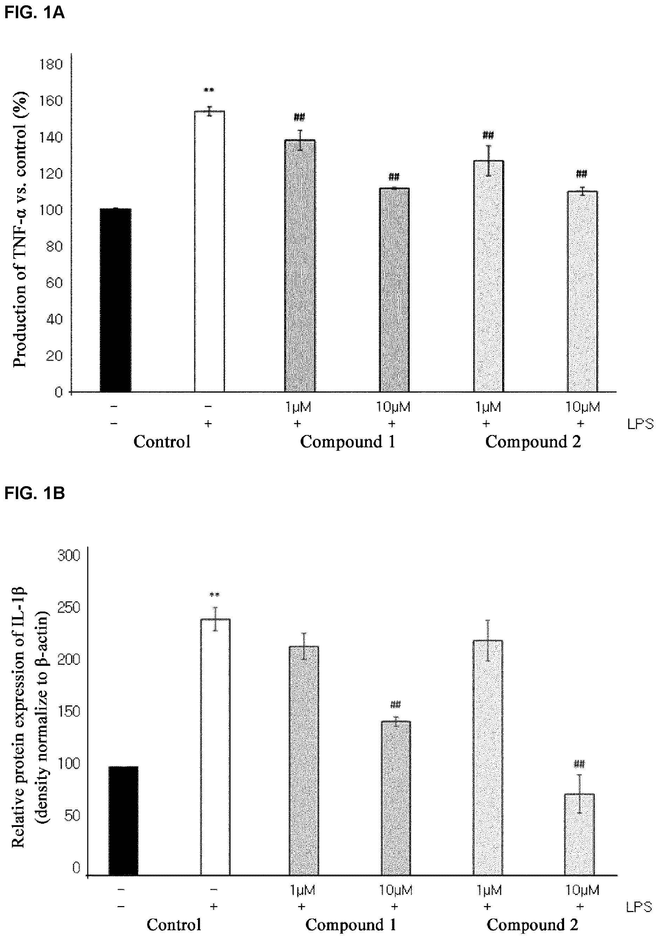

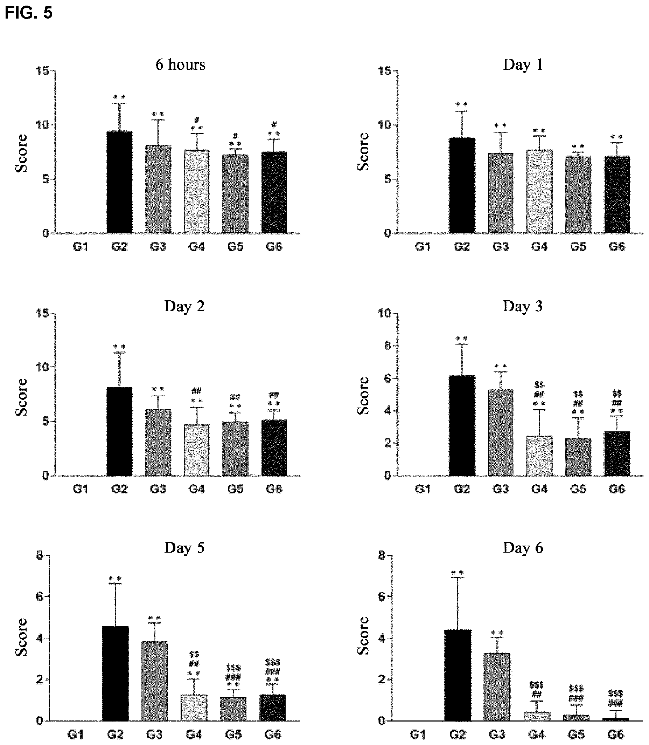

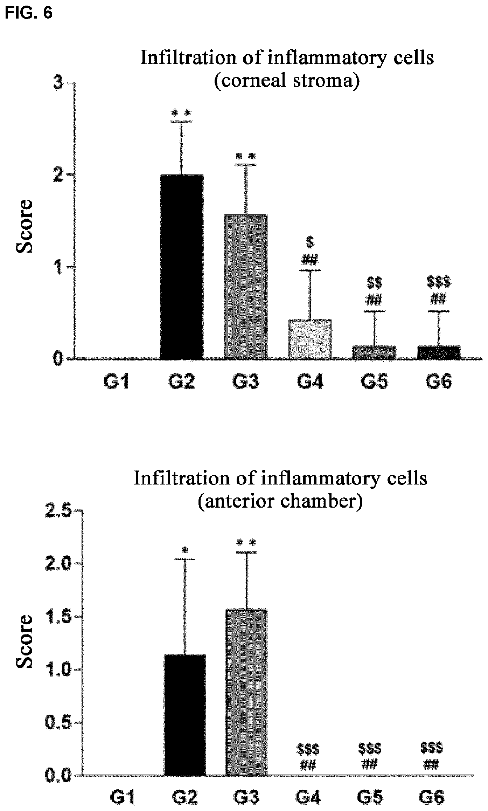

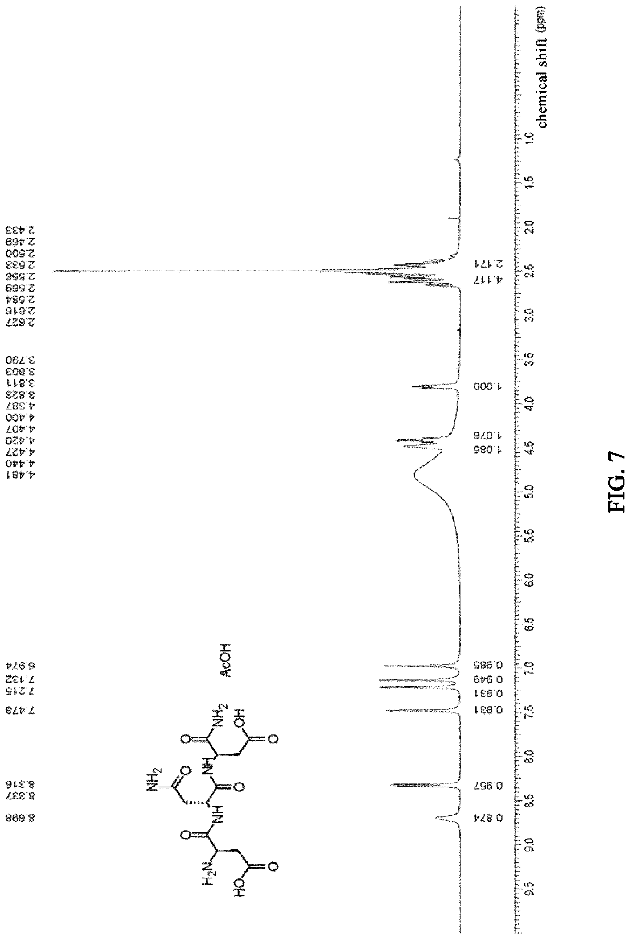

FIGS. 1a and 1b show the result of evaluating the anti-inflammatory activity of the compound of the present disclosure in vitro. FIG. 1a shows the result of evaluating the inhibitory activity of the compound of the present disclosure on the expression of an inflammatory cytokine (TNF-α). FIG. 1b shows the result of evaluating the inhibitory activity of the compound of the present disclosure on the expression of an inflammatory cytokine (IL-1β). In FIG. 1a and FIG. 1b, "Compound 1" represents the compound of Chemical Formula 1a prepared in Example 1, and "Compound 2" represents the acetate of the compound of Chemical Formula 1b prepared in Example 2.

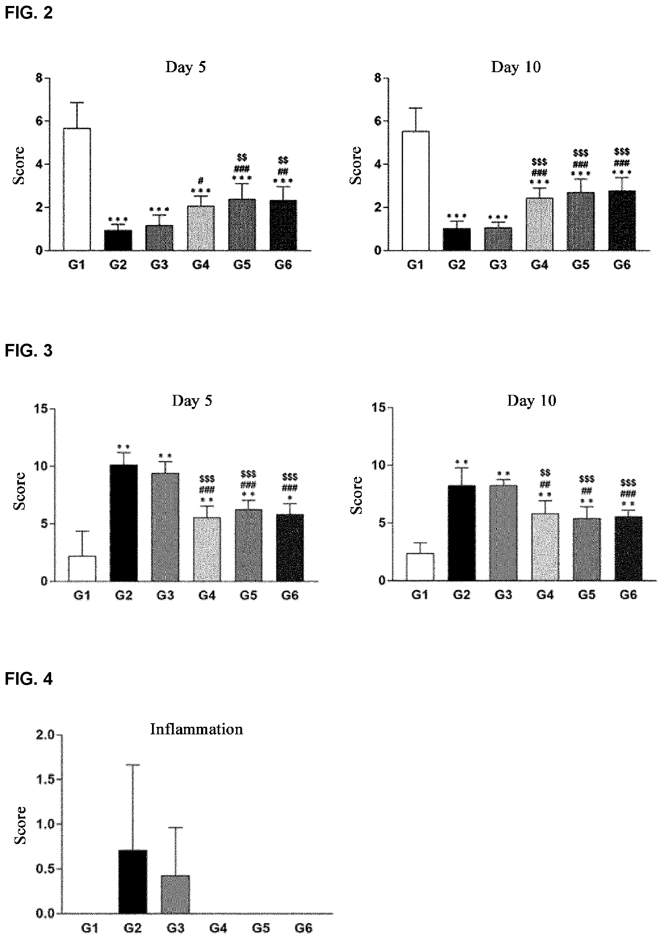

FIG. 2 shows the result of measuring tear volume after instilling the compound of the present disclosure into the eyes of mice in which dry eye syndrome is induced. ***: significant difference with p < 0.001 vs. G1, ###/##/#: significant difference with p < 0.001/p < 0.01/p < 0.05 vs. G2, $$$/$$: significant difference with p < 0.001/p < 0.01 vs. G3.
FIG. 3 shows the result of measuring corneal fluorescein staining scores after instilling the compound of the present disclosure into the eyes of mice in which dry eye syndrome is induced. **/*: significant difference with p < 0.01/p < 0.05 vs. G1, ###/##: significant difference with p < 0.001/p < 0.01 vs. G2, $$$/$$: significant difference with p < 0.001/p < 0.01 vs. G3.
FIG. 4 shows the result of measuring the degree of inflammation through histopathological examination after instilling the compound of the present disclosure into the eyes of mice in which dry eye syndrome is induced.
FIG. 5 shows the result of measuring corneal fluorescein staining scores after instilling the compound of the present disclosure into the eyes of rats in which corneal damage is induced. **: significant difference with p < 0.01 vs. G1, ###/##/#: significant difference with p < 0.001/p < 0.01/p < 0.05 vs. G2, $$$/$$: significant difference with p < 0.001/p < 0.01 vs. G3.
FIG. 6 shows the result of measuring the degree of infiltration of corneal keratocytes and anterior chamber inflammatory cells through histopathological examination after instilling the compound of the present disclosure into the eyes of rats in which corneal damage is induced. **: significant difference with p < 0.01 vs. G1, ##: significant difference with p < 0.01 vs. G2, $$$/$$/$: significant difference with p < 0.001/p < 0.01/p < 0.05 vs. G3.
FIG. 7 shows the ¹H NMR analysis result of the compound of the present disclosure prepared by solution-phase peptide synthesis.

### [Best Mode]

The present disclosure provides a specific D-amino acid-containing peptide derivative, i.e., a compound of Chemical Formula 1, or a pharmaceutically acceptable salt thereof. 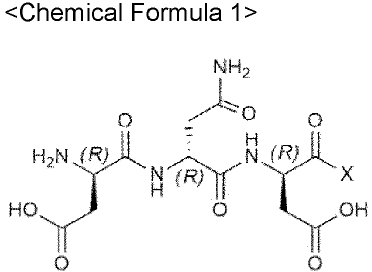 wherein X is -OH or -NH₂.

In an exemplary embodiment, the compound of Chemical Formula 1 or a pharmaceutically acceptable salt thereof of the present disclosure may be in the form of a D-amino acid-containing peptide. In other words, in the compound of Chemical Formula 1 or a pharmaceutically acceptable salt thereof of the present disclosure, X may be -OH and the compound may have the structure of Chemical Formula 1a.

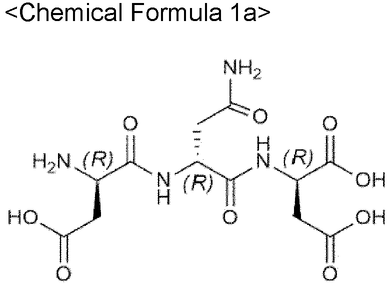

In another exemplary embodiment, the compound of Chemical Formula 1 or a pharmaceutically acceptable salt thereof of the present disclosure may specifically be in the form of a D-amino acid-containing peptide derivative. That is, in the compound of Chemical Formula 1 or a pharmaceutically acceptable salt thereof of the present disclosure, X may be specifically -NH₂ and the compound may have the structure of Chemical Formula 1b.

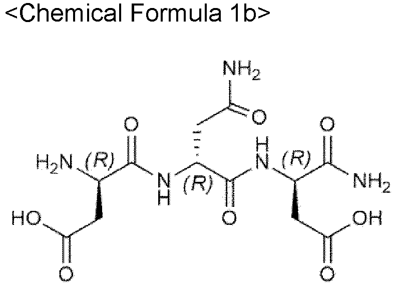

The compound of Chemical Formula 1 of the present disclosure may be in the form of a pharmaceutically acceptable salt. The salt includes common acid addition salts, such as salts derived from inorganic acids, e.g., hydrochloric acid, sulfuric acid, nitric acid, phosphoric acid, perchloric acid bromic acid and salts derived from organic acids, e.g., formic acid, acetic acid, propionic acid, oxalic acid, succinic acid, benzoic acid, citric acid, maleic acid, malonic acid, malic acid, tartaric acid, gluconic acid, lactic acid, gentisic acid, fumaric acid, lactobionic acid, salicylic acid, phthalic acid, embonic acid, aspartic acid, glutamic acid and acetylsalicylic acid. In addition, the salt includes salts of sulfonic acids, such as methanesulfonic acid, ethanesulfonic acid, benzenesulfonic acid and toluenesulfonic acid.

The compound of Chemical Formula 1 according to the present disclosure, or a pharmaceutically acceptable salt thereof, may be prepared by various methods. For example, the compound of Chemical Formula 1 or a pharmaceutically acceptable salt thereof according to the present disclosure may be prepared by solid-phase peptide synthesis or solution-phase peptide synthesis using a D-amino acid [e.g., D-aspartic acid (dASP) or D-asparagine (dASN)] having an amino-protecting group [e.g., Boc (tert-butoxycarbonyl)] and a carboxylic acid-protecting group [e.g., Bzl (benzyl)].

Since the compound of Chemical Formula 1 or a pharmaceutically acceptable salt thereof of the present disclosure exhibits excellent anti-inflammatory activity and also exhibits excellent improvement activity in dry eye syndrome and corneal damage models, it can be usefully used to prevent, ameliorate and treat various ophthalmic diseases. Accordingly, the present disclosure provides a pharmaceutical composition for preventing, ameliorating or treating an ophthalmic disease, which contains a therapeutically effective amount of the compound of Chemical Formula 1, or a pharmaceutically acceptable salt thereof, as an active ingredient.

The present disclosure also provides a therapeutic use of the compound of Chemical Formula 1 or a pharmaceutically acceptable salt thereof. In some aspects, the therapeutic use is a use for preventing or treating an ophthalmic disease.

The present disclosure also provides a method for preventing or treating an ophthalmic disease in a subject in need thereof, which includes administering the pharmaceutical composition containing the compound of Chemical Formula 1, or a pharmaceutically acceptable salt thereof, to the subject.

In this specification, the term "subject" refers to any mammalian subject to which the compound or a pharmaceutically acceptable salt thereof or the composition described in the present specification is administered. Non-limiting examples include human, livestock (e.g., dog, cat, etc.), farm animals (e.g., cattle, sheep, pig, horse, etc.) and laboratory animals (e.g., monkey, rat, mouse, rabbit, guinea pig, etc.) that require diagnosis, management or treatment, especially human. The method described in this specification is applicable to both prevention or treatment of human and veterinary uses.

As used in this specification, the phrase "subject in need thereof" includes a subject such as a mammalian subject to which the administration of the compound or a pharmaceutically acceptable salt thereof or the composition described in the present specification provides benefit.

In this specification, the term "ophthalmic disease" refers to a disease or a condition that affects or relates to the eye, a part of the eye, or an area of the eye. In some aspects, the ophthalmic disease may be one that is caused by the dryness of the eyeball. In some aspects, the ophthalmic disease may be a disease of the cornea or conjunctiva. In some aspects, the ophthalmic disease may be one caused by corneal or conjunctival damage. In some aspects, the ophthalmic disease may be a disease caused by the defect or necrosis of the cornea or conjunctiva. In some aspects, the ophthalmic disease may be a disease caused by the inflammation of the eyeball.

In some aspects, the ophthalmic disease may be one or more diseases selected from the group consisting of dry eye syndrome, ocular inflammation, conjunctival damage, corneal damage and corneal ulcer. In some aspects, the ophthalmic disease may be dry eye syndrome. In some aspects, the ophthalmic disease may be ocular inflammation and may be selected from, for example, conjunctivitis, keratitis (including neurotrophic keratitis), keratoconjunctivitis, uveitis and scleritis. In some aspects, the ophthalmic disease may be conjunctival damage, corneal damage or corneal ulcer.

The pharmaceutical composition of the present disclosure may be prepared in a variety of formulations using pharmaceutically acceptable additives or carriers. Specifically, the pharmaceutical composition of the present disclosure may be in the form of an eye drop formulation, an eye ointment or a spray, and the eye drop formulation may be in the form of a solution or a suspension.

For example, the eye drop formulation in the form of a solution may contain in sterile water, in addition to the compound of the Chemical Formula 1 or a salt thereof, a solubilizing agent such as polyethylene glycol 400, glycerin, etc., a stabilizer such as EDTA, etc., a buffer such as boric acid, etc., and a pH-regulating agent such as hydrochloric acid or sodium hydroxide. In addition, the eye drop formulation in the form of a suspension may contain in sterile water, in addition to the compound of Chemical Formula 1 or a salt thereof, a viscosity-controlling agent such as crosslinked polyvinylpyrrolidone (e.g., povidone K-25), an isotonic agent such as sodium chloride, etc., a stabilizer such as EDTA, etc., a buffer such as boric acid, borax, etc., and a pH-regulating agent such as hydrochloric acid or sodium hydroxide. If necessary, the pharmaceutical composition in the form of the eye drop formulation may be sterilized in accordance with the usual method or may contain an adjuvant such as a preservative, a hydrating agent, an emulsifier, a solubilizer, a salt and/or buffer for osmotic regulation, etc.

The compound of Chemical Formula 1 or a pharmaceutically acceptable salt thereof may be, for example, in the form of an eye drop formulation with a concentration of about 0.01% to about 10%, and may be administered to a patient with an ophthalmic disease at a dosage of 1 to 12 drops per eye in divided doses from 1 to 6 times a day. Of course, the dosage may be changed depending on the patient's age, sex, susceptibility, symptoms or severity of the disease.

The present disclosure also provides a cosmetic composition for preventing or ameliorating an ophthalmic disease, which contains the compound of Chemical Formula 1 or a pharmaceutically acceptable salt thereof. In some aspects, the compound of Chemical Formula 1 or a pharmaceutically acceptable salt thereof may be provided as a cosmetic additive. In some aspects, the cosmetic composition may be a mist or spray formulation. In some aspects, the cosmetic additive may be included in eye cosmetic products such as an eyebrow pencil, an eye liner, an eye shadow, a mascara or eye makeup remover for the purpose of preventing or ameliorating an ophthalmic disease.

Hereafter, the present disclosure will be described in more detail through examples and test examples. However, the following examples and test examples only illustrate the present disclosure, and the present disclosure is not limited by them.

### Example 1. Preparation of compound of Chemical Formula 1a

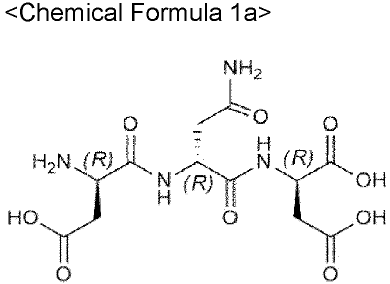

A compound of Chemical Formula 1a was prepared by Fmoc solid-phase peptide synthesis using an automated synthesizer (PeptrEx-R48, Peptron, Daejeon, Korea), using D-aspartic acid (dASP) and D-asparagine (dASN) having Boc as an amino-protecting group and Bzl as a carboxylic acid-protecting group. The Fmoc protecting group was removed by reacting with 20% piperidine in dimethylformamide twice for 10 minutes. Then, coupling was performed using an Fmoc-amino acid (6 eq.), hydroxybenzotriazole (HOBt) (6 eq.), hexafluorophosphate benzotriazole tetramethyl uronium (HBTU) (6 eq.) and N,N-diisopropylethylamine (12 eq.). In each step, the resin was washed twice using dimethylformamide and methanol. The synthesized crude peptide was reacted with a mixture of trifluoroacetic acid (TFA)/1,2-ethanedithiol (EDT)/thioanisole/triisopropylsilane (TIS)/distilled water (DW) (90/2.5/2.5/2.5/2.5 volume) for 2 hours to remove the resin and the amino acid-protecting group. After adding ether to the obtained mixture solution, the peptide was recovered by centrifugation. The crude peptide was dissolved in distilled water and purified by reversed-phase HPLC using a C18 reversed-phase column. The HPLC was performed using a water-acetonitrile linear gradient (acetonitrile concentration: 0-50% (v/v)). The purified fraction was lyophilized, re-dissolved in water containing 0.5% acetic acid and then lyophilized again. The molecular weight of the purified peptide was determined by LC/MS (Shimadzu LC/MS-2020 series, Japan) and lyophilization was conducted using FDT-12012 (Operon, Korea).

### Example 2. Preparation of compound of Chemical Formula 1b

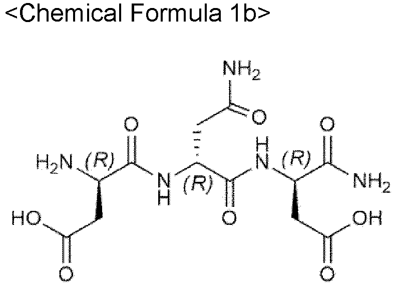

An acetate of the compound of Chemical Formula 1b was prepared by solution-phase peptide synthesis using D-aspartic acid (dASP) and D-asparagine (dASN) having Boc as an amino-protecting group and Bzl as a carboxylic acid-protecting group.

### Step 1:

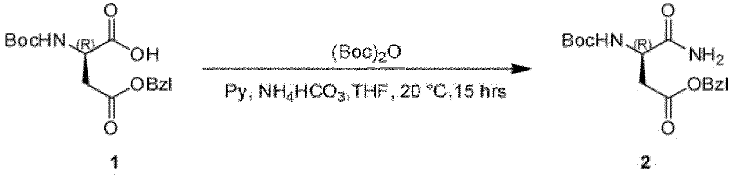

Di-tert-butyl dicarbonate ((Boc)₂O) (43.8 g, 201 mmol, 46.1 mL, 1.30 *eq*.)*,* pyridine (Py) (12.2 g, 154 mmol, 12.4 mL, 1.00 eq.) and NH₄HCO₃ (18.3 g, 231 mmol, 19.1 mL, 1.50 eq.) were added to a solution of Compound 1 (50.0 g, 154 mmol, 1.00 eq.) in tetrahydrofuran (500 mL). The mixture was stirred at 20 °C for 15 hours. As a result of TLC analysis (petroleum ether:ethyl acetate = 1:3), it was confirmed that Compound 1 (R_{f} = 0.52) was consumed completely and a new spot (R_{f} = 0.45) was formed. The reaction mixture was diluted with H₂O (500 mL) and vacuum-filtered to obtain a residue. The crude product was triturated with H₂O (300 mL) at 20 °C for 0.5 hour and then filtered to obtain a white solid, which was treated with petroleum ether (100 mL) for 0.5 hours at 20 °C. Compound 2 (47.0 g, 143 mmol, 92.6% yield, 98.3% purity) was obtained as a white solid, which was identified by ¹H NMR & LCMS (Rt = 1.08 min, MS cal.: 322.1, MS observed: [M+H]⁺ = 323.0). The result of the¹H NMR analysis was as follows.

¹H NMR (400 MHz, DMSO-d₆) δ 7.37-7.31 (m, 5H), 7.25 (s, 1H), 7.09-7.01 (m, 2H), 5.12-5.05 (m, 2H), 4.32-4.27 (m, 1H), 2.79-2.74 (m, 1H), 2.61-2.57 (m, 1H), 1.37 (s, 9H).

### Step 2:

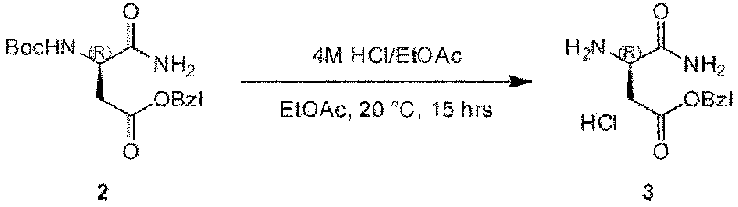

HCl/EtOAc (4 M, 196 mL, 5.49 eq.) was added to a solution of Compound 2 (47.0 g, 143 mmol, 98.3% purity, 1.00 eq.) in ethyl acetate (EtOAc) (100 mL). The mixture was stirred at 20 °C for 15 hours. As a result of TLC analysis (petroleum ether:ethyl acetate = 1:3), it was confirmed that Compound 2 (R_{f} = 0.45) was consumed completely and a new spot (R_{f} = 0.18) was formed. The reaction mixture was filtered to obtain a residue, which was treated with EtOAc (100 mL) at 20 °C for 0.5 hours. Compound 3 (37.0 g, 141 mmol, 98.4% yield, 98.7% purity, HCl salt) was obtained as a white solid, which was identified by ¹H NMR & LCMS (Rt = 0.14 min, MS cal.: 222.1, MS observed: [M+H]⁺ = 223.1), and HPLC (Rt = 1.63 min, 98.7% purity). The result of the ¹H NMR analysis was as follows.

¹H NMR (400 MHz, D₂O) δ 7.41 (s, 5H), 5.20 (s, 2H), 4.37-4.34 (m, 1H), 3.16-3.03 (m, 2H).

### Step 3:

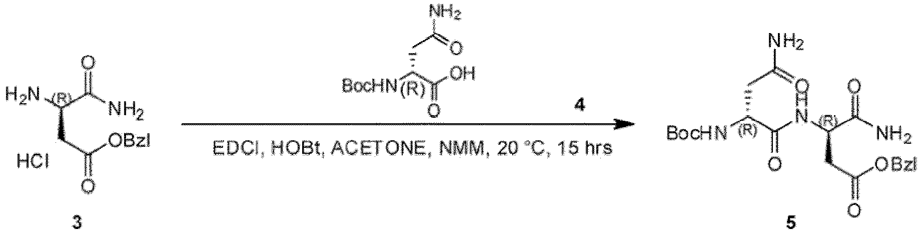

After adding 1-ethyl-3-(3-dimethylaminopropyl)carbodiimide (EDCI), (25.8 g, 135 mmol, 1.10 eq.), hydroxybenzotriazole (HOBt) (16.5 g, 122 mmol, 1.00 eq.) and Compound 3 (36.9 g, 141 mmol, 98.7% purity, 1.15 eq., HCl salt) to a solution of Compound 4 (28.5 g, 122 mmol, 1.00 eq.) in acetone (500 mL) at 20 °C, N-methylmorpholine (NMM) (37.2 g, 368 mmol, 40.4 mL, 3.00 eq.) was added dropwise at 20 °C. A solid was precipitated 2 hours later. The obtained mixture was stirred at 20 °C for 13 hours. As a result of TLC analysis (dichloromethane:methanol = 7:1), it was confirmed Compound 3 (R_{f} = 0.24) was consumed completely and a new spot (R_{f} = 0.11, 0.35, 0.41, 0.90) was formed. The reaction mixture was diluted with H₂O (1000 mL) and filtered to obtain a residue. The crude product was treated with H₂O (400 mL x 3) at 20 °C for 0.5 hour and filtered to obtain a white solid, which was treated with petroleum ether (300 mL) for 0.5 hours at 20 °C. Compound 5 (40.6 g, 92.0 mmol, 74.9% yield, 98.9% purity) was obtained as a white solid, which was identified by ¹H NMR & LCMS (Rt = 0.47 min, MS cal.: 436.2, MS observed: [M+H]⁺ = 437.0) and HPLC (Rt = 1.77 min, 98.9% purity). The result of the ¹H NMR analysis was as follows.

¹H NMR (400 MHz, DMSO-d₆) δ 8.19 (d, *J* = 8.4 Hz, 1H), 7.39-7.29 (m, 7H), 7.21 (s, 1H), 5.07 (s, 2H), 4.60-4.55 (m, 1H), 4.19-4.14 (m, 1H), 2.87 -2.81 (m, 1H), 2.68-2.62 (m, 1H), 2.55-2.53 (m, 1H), 2.42-2.36 (m, 1H), 1.36 (s, 9H).

### Step 4:

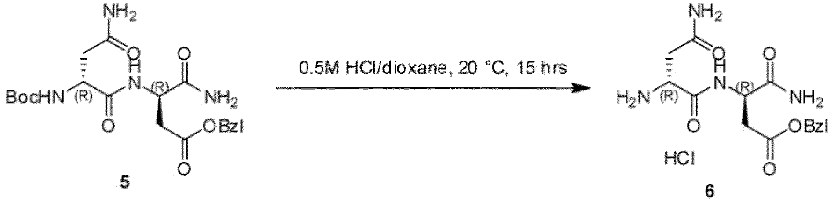

A mixture of Compound 5 (40.6 g, 92.0 mmol, 98.9% purity, 1.00 eq.) in HCl/dioxane (0.50 M, 552 mL, 3.00 eq.) was stirred at 20 °C for 15 hours. As a result of TLC analysis (dichloromethane:methanol = 7:1), it was confirmed Compound 5 (R_{f} = 0.57) was consumed completely and a new spot (R_{f} = 0.07) was formed. The reaction mixture was diluted with methyl tert-butyl ether (MTBE) (500 mL). After a solid was precipitated, it was vacuum-filtered to obtain a white solid. Compound 6 (35.4 g, crude product, HCl salt) was obtained as a white solid, which was identified by ¹H NMR & LCMS (Rt = 0.12 min, MS cal.: 336.1, MS observed: [M+H]⁺ = 337.1). The result of the ¹H NMR analysis was as follows.

¹H NMR (400 MHz, DMSO-d₆) δ 7.32-7.25 (m, 5H), 5.04 (s, 2H), 4.59-4.55 (m, 1H), 4.04-4.01 (t, *J* = 6.8 Hz, 1H), 2.80-2.70 (m, 4H).

### Step 5:

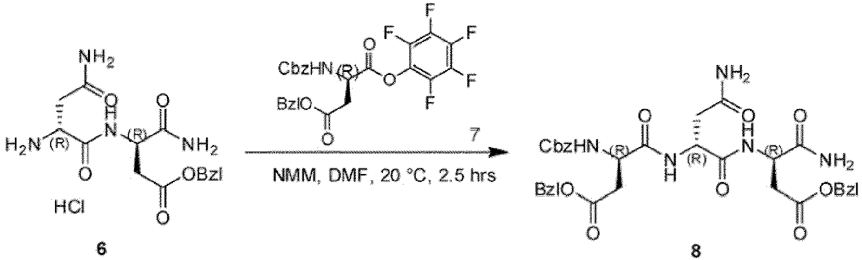

N-methylmorpholine (NMM) (11.9 g, 118 mmol, 13.0 mL, 3.00 *eq*.) was added to a solution of Compound 7 (21.0 g, 39.4 mmol, 98.2% purity, 1.00 *eq*.) and Compound 6 (14.6 g, 39.4 mmol, 1.00 *eq*. HCl salt) in dimethylformamide (100 mL). The mixture was stirred at 20 °C for 2.5 hours. As a result of LCMS analysis, it was confirmed that Compound 7 was consumed completely and one major peak corresponding to the target mass was detected (Rt = 0.34 min, MS cal.: 675.2, MS observed: [M+H]⁺ = 676.1). The reaction mixture was diluted with H₂O (500 mL) and ethyl acetate (150 mL). After a solid was precipitated, it was stirred at 20 °C for 3 hours and vacuum-filtered to obtain a yellow solid, which was treated with H₂O (150 mL x 3) at 20 °C for 1 hour. Compound 8 (22.0 g, crude product) was obtained as a yellow solid, which was identified by LCMS (Rt = 0.58 min, MS cal.: 675.2, MS observed: [M+H]⁺ = 676.3).

### Step 6:

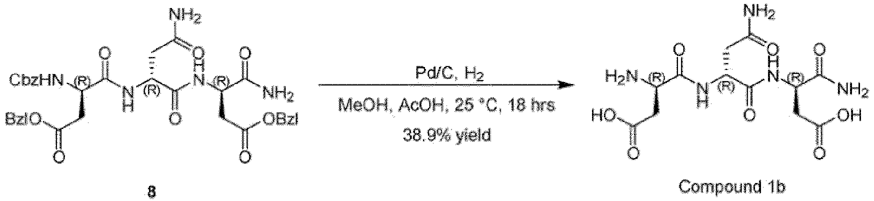

Pd/C (10.0% purity, 4.80 g) was added to a solution of Compound 8 (24.0 g, 35.5 mmol, 1.00 eq.) in methanol (350 mL) and acetic acid (350 mL) under nitrogen atmosphere. The resulting suspension was degassed and purged with hydrogen 3 times. The mixture was stirred under hydrogen (50 psi) at 25 °C for 18 hours. As a result of LCMS analysis, it was confirmed that Compound 8 was consumed completely and one major peak corresponding to the target mass was detected (Rt = 0.07 min, MS cal.: 361.1, MS observed: [M+H]⁺ = 362.1). The mixture was filtered and the resulting filter cake was washed with H₂O (50.0 mL x 2). After combining the filtrate, a residue was obtained by vacuum drying. The crude product was treated with ethanol (50.0 mL) for 0.6 hours at 20 °C and a yellow solid was obtained by vacuum filtration, which was treated with methanol (30.0 mL) at 20 °C for 0.6 hours. The product (10.0 g, crude product) was obtained as a yellow solid, which was identified by LCMS (Rt = 0.10 min, MS cal.: 361.1, MS observed: [M+H]⁺ = 361.9). The crude product was purified by prep-HPLC (0.5% HOAc) to obtain the product (5.00 g, 11.5 mmol, 97.4% purity, HOAc salt) as a white solid, which was identified by special LCMS (Rt = 2.87 min, MS cal.: 361.1, MS observed: [M+H]⁺ = 362.1) and ¹H NMR. The result of the ¹H NMR analysis (FIG. 7) is as follows.

¹H NMR (400 MHz, DMSO-d₆) δ 8.69 (s, 1H), 8.32 (d, *J* = 8.4 Hz, 1H), 7.47 (s, 1H), 7.21 (s, 1H), 7.13 (s, 1H), 6.97 (s, 1H), 4.48 (s, 1H), 4.41 (dt, *J*₁ = 5.2 Hz, *J*₂ = 8.0 Hz, 1H), 3.81 (dd, *J*₁ = 5.2 Hz, *J*₂ =8.0 Hz, 1H), 2.69-2.52 (m, 4H), 2.46-2.37 (m, 2H).

### Example 1: In-vitro anti-inflammatory test

Lipopolysaccharide (LPS)-treated macrophages (RAW 264.7) were treated with the compounds prepared in Examples 1 and 2 as test substances, respectively, and the change in the production of inflammatory cytokines (TNF-α and IL-1β) was measured by ELISA and western blotting, respectively.

Specifically, 1x10⁴ macrophages (RAW 264.7) were dispensed in each well of a 96-well plate together with 0.1 mL of Dulbecco's modified Eagle's medium (DMEM) containing 10% FBS (GIBCO) and 1% penicillin/streptomycin. After incubation for 1 day in a 5% CO₂ incubator at 37 °C, when the confluency of the cells reached 80% or higher, LPS (100 ng/mL) and the test substance were treated at concentrations of 1 µM and 10 µM, respectively, and incubated for 24 hours. A control group was treated only with LPS (100 ng/mL) and incubated for 24 hours. The supernatant of each test group was prepared as a sample for ELISA analysis. The production of TNF-α was investigated using an ELISA assay kit (ELISA MAX Standard Set Human TNF-α; BioLegend, #430201) and a microplate reader at 450 nm according to the manufacturer's protocol. The result is shown in FIG. 1a.

In addition, 5x10⁶ macrophages (RAW 264.7) were dispensed in each well of a 96-well plate together with 0.1 mL of Dulbecco's modified Eagle's medium (DMEM) containing 10% FBS (GIBCO) and 1% penicillin/streptomycin. After incubation for 1 day in a 5% CO₂ incubator at 37 °C, when the confluency of the cells reached 80% or higher, LPS (100 ng/mL) and the test substance were treated at concentrations of 1 µM and 10 µM, respectively, and incubated for 30 minutes. A control group was treated only with LPS (100 ng/mL) and incubated for 30 minutes. The cells were lysed using an NP40 cell lysis buffer and quantified by Bradford assay. Then, the expression level of IL-1β was measured by western blotting. The result is shown in FIG. 1b.

As can be seen from FIG. 1a and FIG. 1b, the control group showed increased secretion and expression of inflammatory cytokines (TNF-α and IL-1β) after treatment with LPS. On the other hand, it can be seen that the secretion and expression of the inflammatory cytokines (TNF-α and IL-1β) were inhibited in a concentration-dependent manner in the test groups treated with the test substances. Accordingly, since the compounds of Chemical Formula 1a and Chemical Formula 1b obtained according to the present disclosure exhibit excellent anti-inflammatory activity, they can be usefully used for amelioration of ocular inflammation such as conjunctivitis, keratitis, keratoconjunctivitis, uveitis, scleritis, etc.

### Test Example 2: Assessment of activity in dry eye syndrome model

### 1. Test methods

### (1) Administration of test substance

Dry eye syndrome was induced in specific pathogen-free (SPF) C57BL/6NHsd mice (male, 7 weeks old) [Koatech, Korea]. Specifically, during the dry eye syndrome induction period (from day -10 to day 0), scopolamine hydrobromide dissolved in physiological saline (PBS) was injected subcutaneously into the back skin of mice at a dose of 0.5 mg/0.2 mL, 4 times a day, for 10 days. After measuring the tear volume of the dry eye syndrome-induced mice, the mice were randomly divided into groups of G2 to G6 as shown in Table 1 so that the average tear volume of each group was distributed uniformly. G1 is a normal control group in which dry eye syndrome was not induced.

From the day of the induction of dry eye syndrome (day 0), the compound prepared in Example 2 as a test substance (i.e., acetate of the compound of the Chemical Formula 1b, hereinafter referred to as 'Compound 1b') was dissolved in physiological saline (PBS) at concentrations of 0.1% and 1% and dropped onto the center of the right cornea of the mice of G4 and G5 3 times a day for 10 days, respectively. G2 is a dry eye syndrome-induced control group. For G3, physiological saline (PBS) was dropped onto the center of the cornea of the right eye of the mice in which dry eye syndrome was induced. For G6 (positive control group), Restasis^{®} ophthalmic solution (0.05%, containing cyclosporine) was dropped onto the center of the cornea of the right eye of the dry eye syndrome-induced mice.

**[Table 1]**

| Group | Sex | Number of animals (n) | Animal No. | Induction of dry eye syndrome | Test substance | Dosage | Application amount (µL/eye) |
|---|---|---|---|---|---|---|---|
| G1 | Male | 5 | 1-5 | No | - | - | - |
| G2 | Male | 7 | 6-12 | Yes | - | - | - |
| G3 | Male | 7 | 13-19 | Yes | PBS | - | 2 |
| G4 | Male | 7 | 20-26 | Yes | Compound 1b | 0.1% | 2 |
| G5 | Male | 7 | 27-33 | Yes | Compound 1b | 1% | 2 |
| G6 | Male | 7 | 34-40 | Yes | Restasis^{®} | 0.05% | 2 |

| | | | | | | | |
|---|---|---|---|---|---|---|---|
| G1: normal control, G2: induced control, G6: positive control | | | | | | | |

### (2) Measurement of tear volume and corneal fluorescein staining score

Tear volume was measured by phenol red thread (PRT) test. Specifically, on days 5 and 10 after the start of the administration of the test substance, a phenol red thread was placed in the lateral canthus of the eye for 20 seconds and then tear volume (mm) was measured.

After measuring the tear volume, the animals were anesthetized on days 5 and 10 day after the start of the administration of the test substance, and 1 µL of 1% sodium fluorescein was instilled into the lower conjunctival sac for the cornea of the right eye of the animal, and the degree of penetration into the 5 corneal zones (superior, nasal, central, inferior and temporal) was evaluated according to the following criteria of the National Eye Institute (NEI) grading system using cobalt blue light.
- Scores: 0-3 (0: normal, 1: mild, 2: moderate, 3: severe)
- Total score: 15

### (3) Autopsy and histopathological examination

On the day of autopsy (day 10), the animal was euthanized and the eyeball was removed to include the conjunctiva and fixed in a 10% neutral buffered formalin solution. Samples for histopathological examination were prepared through the processes of trimming, dehydration, paraffin embedding and thin-sectioning of the fixed tissue, followed by hematoxylin and eosin (H&E) staining. Then, the infiltration of inflammatory cells into the cornea, conjunctiva and anterior chamber was evaluated by optical microscopy (Olympus BX53, Japan). The inflammation of the cornea and conjunctiva was assessed according to the known method. The scoring criteria were as follows. Scores: 0 (normal), 1 (mild), 2 (moderate), 3 (severe).

### (4) Statistical analysis

Statistical analysis was performed using Prism 7.04 (GraphPad Software Inc., San Diego, CA, USA), and a p-value smaller than 0.05 was judged to be statistically significant.

### 2. Test results

### (1) Measurement of tear volume

The tear volume measurement result is shown in FIG. 2. On days 5 and 10 after the start of the administration of the test substance, the tear volume levels of the induced control group (G2), PBS group (G3), 0.1% test substance-administered group (G4), 1% test substance-administered group (G5) and positive control group (G6) were statistically significantly lower than that of the normal control group (G1) (p < 0.001). The tear volume levels of G4, G5 and G6 were significantly higher than that of G2 (p < 0.001, p < 0.01 or p < 0.05). On day 5 after the start of the administration of the test substance, the tear volume levels of G5 and G6 were statistically significantly higher than that of G3 (p < 0.01). On day 10 after the start of the administration of the test substance, the tear volume levels of G4, G5 and G6 were significantly higher than that of G3 (p < 0.001).

### (2) Measurement of corneal fluorescein staining score

The corneal fluorescein staining score measurement result is shown in FIG. 3. From day 5 to day 10 after the start of the administration of the test substance, the levels of corneal fluorescein staining scores in G2, G3, G4, G5 and G6 were statistically significantly higher than that of G1 (p < 0.01 or p < 0.05). The levels of corneal fluorescein staining scores in G4, G5, and G6 were significantly lower than that of G2, and the levels of corneal fluorescein staining scores in G4, G5, and G6 were statistically significantly lower than that of G3 (p < 0.001 or p < 0.01).

### (3) Histopathological examination

The histopathological examination result is shown in FIG. 4. The level of inflammation in G2 and G3 was statistically significantly higher as compared to G1 (p < 0.01), and the level of inflammation and fibrosis in G4, G5 and G6 was significantly lower as compared to G2 and G3 (p < 0.001 or p < 0.05).

### 3. Review

The effect of the test substance on a dry eye syndrome-induced C57BL/6NHsd mouse model was evaluated (G1: normal control, G2: induced control, G3: PBS-administered group, G4: 0.1% test substance-administered group, G5: 1% test substance-administered group, G6: positive control group).

As a result of measuring the tear volume using a phenol red thread, the tear volume level of the 0.1% test substance-administered group and 1% test substance-administered group was statistically significantly higher than that of the induced control group from day 5 to day 10 after the start of the administration of the test substance, and the tear volume level of the 1% test substance-administered group was significantly higher than that of the PBS group.

As a result of the measuring the corneal fluorescein staining score, the level of corneal fluorescein staining score in the 0.1% test substance-administered group and the 1% test substance-administered group was observed to be statistically significantly lower than that of the induced control group and the PBS-administered group from day 5 to day 10 after the start of the administration of the test substance.

As a result of the histopathological examination, no inflammation was observed in the cornea and conjunctiva in the 0.1% test substance-administered group and the 1% test substance-administered group.

Therefore, repeated instillation of the test substance in the dry eye syndrome-induced C57BL/6 mouse model may be useful in ameliorating dry eye syndrome and ocular inflammation.

### Example 3: Evaluation of activity in corneal damage model

### 1. Test methods

### (1) Administration of test substance

Corneal damage was induced in pathogen-free (SPF) Sprague-Dawley rats (male, 7 weeks old) [Koatech, Korea]. Specifically, after anesthetizing the rats, a filter paper soaked in 2 µL of 0.1 M NaOH solution was applied to the center of the cornea of the right eye for about 10 seconds, and then washed with physiological saline. The body weight of the corneal damage-induced rats was measured, and they were randomly divided into groups G2 to G6 as shown in Table 2 so that the average body weight of each group was distributed uniformly. G1 is a normal control group in which corneal damage was not induced.

From the day of the corneal damage (day 0), the compound prepared in Example 2 as a test substance (i.e., acetate of the compound of the Chemical Formula 1b, hereinafter referred to as 'Compound 1b') was dissolved in physiological saline (PBS) at concentrations of 0.1% and 1% and instilled into the eye of the mice of G4 and G5 at 10 µL/eye, 3 times a day, for 7 days. G2 is an induced control group in which corneal damage was induced. For G3, physiological saline (PBS) was instilled into the eye of the corneal damage-induced rats. For G6 (positive control), Restasis^{®} ophthalmic solution (0.05%, containing cyclosporine) was instilled into the eye of the corneal damage-induced rats.

**[Table 2]**

| Group | Sex | Number of animals (n) | Animal No. | Induction of corneal damage | Test substance | Dosage | Application amount (µL/eye) |
|---|---|---|---|---|---|---|---|
| G1 | Male | 5 | 1-5 | No | - | - | - |
| G2 | Male | 7 | 6-12 | Yes | - | - | - |
| G3 | Male | 7 | 13-19 | Yes | PBS | - | 10 |
| G4 | Male | 7 | 20-26 | Yes | Compound 1b | 0.1% | 10 |
| G5 | Male | 7 | 27-33 | Yes | Compound 1b | 1% | 10 |
| G6 | Male | 7 | 34-40 | Yes | Restasis^{®} | 0.05% | 10 |

| | | | | | | | |
|---|---|---|---|---|---|---|---|
| G1: normal control, G2: induced control, G6: positive control | | | | | | | |

### (2) Measurement of corneal fluorescein staining score

The animals were anesthetized at 6 hours or on days 1, 2, 3, 5 and 7 after the induction of corneal damage, and 1 µL of 1% sodium fluorescein was instilled into the lower conjunctival sac for the cornea of the right eye of the animal, and the degree of penetration into the 5 corneal zones (superior, nasal, central, inferior and temporal) was evaluated according to the following criteria of the National Eye Institute (NEI) grading system using cobalt blue light.
- Scores: 0-3 (0: normal, 1: mild, 2: moderate, 3: severe)
- Total score: 15

### (3) Autopsy and histopathological examination

On the day of autopsy (day 7), the animal was euthanized, and the eyeball was removed to include the conjunctiva and fixed in a 10% neutral buffered formalin solution. Samples for histopathological examination were prepared through the processes of trimming, dehydration, paraffin embedding and thin-sectioning of the fixed tissue, followed by hematoxylin and eosin (H&E) staining. Then, the infiltration of inflammatory cells into the cornea, conjunctiva and anterior chamber was evaluated by optical microscopy (Olympus BX53, Japan). The infiltration of inflammatory cells into the corneal stroma and anterior chamber was evaluated according to the known method. The scoring criteria were as follows. Scores: 0 (normal), 1 (mild), 2 (moderate), 3 (severe).

### (4) Statistical analysis

Statistical analysis was performed using Prism 7.04 (GraphPad Software Inc., San Diego, CA, USA), and a p-value smaller than 0.05 was judged to be statistically significant.

### 2. Test results

### (1) Measurement of corneal fluorescein staining score

The corneal fluorescein staining score measurement result is shown in FIG. 5. From 6 hours to 5 days after the start of the administration of the test substance, the level of corneal fluorescein staining scores in all the corneal damage groups (G2-G6) was statistically significantly higher than that of the normal control group (G1) (p < 0.01). On day 6 after the start of the administration of the test substance, the level of corneal fluorescein staining scores in the induced control group (G2) and the PBS-administered group (G3) was significantly higher than that of G1 (p < 0.01). At 6 hours and on days 2 to 6 after the start of test substance, the level of corneal fluorescein staining scores in the 0.1% test substance-administered group (G4), 1% test substance-administered group (G5) and positive control group (G6) was significantly lower than that of G2 (p < 0.001, p < 0.01 or p < 0.05). On days 3 to 6 after the start of test substance, the level of corneal fluorescein staining scores in G4, G5 and G6 was significantly lower than that of G3 (p < 0.001 or p < 0.01).

### (2) Histopathological examination

The histopathological examination result is shown in FIG. 6. The degree of infiltration of inflammatory cells into corneal keratocytes and anterior chamber was statistically significantly higher in G2 and G3 than that of G1 (p < 0.01 or p < 0.05), and the degree of infiltration of inflammatory cells into corneal keratocytes and anterior chamber was statistically significantly lower in G4, G5 and G6 than that of G2 and G3 (p < 0.001, p < 0.01 or p < 0.01).

### 3. Review

The effect of the test substance on a corneal damage-induced Sprague-Dawley rat model was evaluated (G1: normal control, G2: induced control, G3: PBS-administered group, G4: 0.1% test substance-administered group, G5: 1% test substance-administered group, G6: positive control group).

As a result of measuring the corneal fluorescein staining score, the level of corneal fluorescein staining score in the 0.1% test substance-administered group and the 1% test substance-administered group was significantly lower than that of the induced control group during all the test periods except day 1 after the start of administration of the test substance, and the level of corneal fluorescein staining score in the 0.1% test substance-administered group and the 1% test substance-administered group was observed to be significantly lower than that of the PBS-administered group from day 3 after the start of the test substance until the end of the test.

As a result of the histopathological examination, it was observed that the scores of inflammatory cell infiltration into the corneal stroma and anterior chamber of the eye were statistically significantly lower in the 0.1% test substance-administered group and the 1% test substance-administered group as compared to the induced control group and the PBS-administered group. The result of the histopathological examination was consistent with the result of the corneal fluorescein staining score measurement.

Therefore, repeated instillation of the test substance in the corneal damage-induced Sprague-Dawley rat model may be useful for conjunctival damage, corneal damage or corneal ulcer. In addition, repeated instillation of the test substance may be useful for ameliorating ocular inflammation (e.g., conjunctivitis, keratitis, keratoconjunctivitis, neurotrophic keratitis, etc.) caused by the above diseases.

## Claims

1. A compound of Chemical Formula 1 or a pharmaceutically acceptable salt thereof: 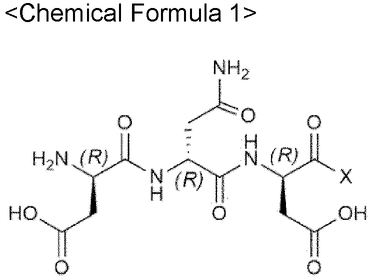 wherein X is -OH or -NH₂.

2. The compound or pharmaceutically acceptable salt thereof according to claim 1, wherein X is -NH₂.

3. A pharmaceutical composition for preventing, ameliorating or treating an ophthalmic disease, comprising the compound or pharmaceutically acceptable salt thereof according to claim 1 or 2 as an active ingredient.

4. The pharmaceutical composition according to claim 3, wherein the ophthalmic disease is one or more diseases selected from a group consisting of dry eye syndrome, ocular inflammation, conjunctival damage, corneal damage and corneal ulcer.

5. The pharmaceutical composition according to claim 3, wherein the ophthalmic disease is dry eye syndrome.

6. The pharmaceutical composition according to claim 3, wherein the ophthalmic disease is ocular inflammation.

7. The pharmaceutical composition according to claim 3, wherein the ophthalmic disease is conjunctival damage, corneal damage or corneal ulcer.

8. The pharmaceutical composition according to claim 3, which is in the form of an eye drop formulation, an eye ointment or a spray.

9. The pharmaceutical composition according to claim 8, wherein the eye drop formulation is in the form of a solution or a suspension.

10. A cosmetic composition for preventing or ameliorating an ophthalmic disease, comprising the compound or pharmaceutically acceptable salt thereof according to claim 1 or 2 as an active ingredient.

11. A therapeutic use of the compound or pharmaceutically acceptable salt thereof according to claim 1 or 2.

12. Use of the compound or pharmaceutically acceptable salt thereof according to claim 1 or 2 for prevention or treatment of an ophthalmic disease.

13. A method for preventing or treating an ophthalmic disease in a subject in need thereof, comprising administering the compound or pharmaceutically acceptable salt thereof according to claim 1 or 2 to the subject.
